# EUROPÄISCHE PATENTSCHRIFT

(11) **EP 3 024 511 B1**
(45) Veröffentlichungstag und Bekanntmachung des Hinweises auf die Patenterteilung: **30.05.2018**
(21) Anmeldenummer: 14741614.3
(22) Anmeldetag: 18.07.2014
(51) Int. Cl.: A61M 1/16, A61M 1/36

(54) **EXTRAKORPORALE BLUTBEHANDLUNGSVORRICHTUNG**
EXTRACORPOREAL BLOOD TREATMENT APPARATUS
DISPOSITIF DE TRAITEMENT EXTRACORPOREL DU SANG

(30) Priorität: 25.07.2013 DE 102013012366
(43) Veröffentlichungstag der Anmeldung: 01.06.2016
(73) Patentinhaber: Fresenius Medical Care Deutschland GmbH, 61352 Bad Homburg (DE)
(72) Erfinder: WIKTOR, Christoph, 63571 Gelnhausen (DE)
(74) Vertreter: Oppermann, Frank
(86) Internationale Anmeldenummer: PCT/EP2014/065558
(87) Internationale Veröffentlichungsnummer: WO 2015/011063

(56) Entgegenhaltungen:
- EP-A2- 1 086 712
- DE-A1-102010 035 498

## Beschreibung

Die Erfindung betrifft eine extrakorporale Blutbehandlungsvorrichtung mit einem Flüssigkeitssystem und einer Pumpe zum Fördern von Flüssigkeit in dem Flüssigkeitssystem, wobei die Pumpe eine nicht-okkludierende Pumpe ist.

Es sind verschiedene Verfahren zur extrakorporalen Blutbehandlung bekannt, beispielsweise die Hämodialyse, die Hämofiltration sowie die Kombination aus beiden Verfahren, die als Hämodiafiltration bezeichnet wird. Die extrakorporalen Blutbehandlungsvorrichtungen verfügen über eine Blutbehandlungseinheit, die durch eine semipermeable Membran in eine Blutkammer und eine Dialysierflüssigkeitskammer unterteilt ist. Während der Blutbehandlung strömt das Blut des Patienten in einem extrakorporalen Blutkreislauf durch die Blutkammer des Dialysators, während Dialysierflüssigkeit in einem Dialysierflüssigkeitssystem durch die Dialysierflüssigkeitskammer des Dialysators strömt. Der extrakorporale Blutkreislauf und das Dialysierflüssigkeitssystem werden nachfolgend allgemein als Flüssigkeitssystem der extrakorporalen Blutbehandlungsvorrichtung bezeichnet.

Zum Fördern der Flüssigkeiten im Flüssigkeitssystem finden bei den extrakorporalen Blutbehandlungsvorrichtungen im Allgemeinen peristaltische Pumpen Verwendung, bei denen sich mindestens ein Verschlusskörper längs der als Pumpenraum dienenden elastischen Schlauchleitung bewegt. Diese Pumpen werden daher auch als okkludierende Schlauchpumpen bezeichnet. Die gebräuchlichste okkludierende Schlauchpumpe ist eine Rollenpumpe, in die ein Abschnitt der Schlauchleitung eingelegt wird.

Es sind aber auch extrakorporale Blutbehandlungsvorrichtungen bekannt, bei denen anstelle von peristaltischen Pumpen nicht-okkludierende Pumpen, beispielsweise Zentrifugal- oder Impellerpumpen verwendet werden, die nicht über einen Verschlusskörper verfügen, mit dem die Schlauchleitung okkludiert wird.

Das Ausschalten von okkludierenden Pumpen oder das Reduzieren der Drehzahl dieser Pumpen im Flüssigkeitssystem der extrakorporalen Blutbehandlungsvorrichtung erweist sich in der Praxis als unproblematisch, da aufgrund des Verschlusskörpers nicht die Gefahr des Rückflusses der Flüssigkeiten besteht. Bei nicht-okkludierenden Pumpen hingegen besteht die Gefahr, dass Flüssigkeit, insbesondere Blut des Patienten, beim Abschalten oder Reduzieren der Drehzahl der Pumpe entgegen der Förderrichtung strömt.

Die DE 10 2010 035 498 A1 beschreibt ein Verfahren zur Erkennung einer Leckage im extrakorporalen Blutkreislauf einer Blutbehandlungsvorrichtung, die über eine venöse Absperreinrichtung verfügt. Um eine potentielle Leckage zu detektieren, werden zunächst die Absperreinrichtung sowie die Ventile im Dialysatkreislauf geschlossen. Zur Detektion eines Blutlecks werden optische Messungen für zwei verschiedene Förderzustände durchgeführt. Hierfür wird die Fördereinrichtung für eine bestimmte Zeit gestoppt und nach einer unbestimmten oder vorbestimmten, jedenfalls aber ausreichenden Zeit wird die Fördereinrichtung wieder gestartet. Wenn die Fördereinrichtung eine nicht-okkludierende Blutpumpe sein sollte, wäre bei einer nicht vorhandenen oder geöffneten Absperreinrichtung in der Zuführleitung ein Rückfluss von Blut zu dem Patienten zu befürchten.

Der Erfindung liegt die Aufgabe zu Grunde, eine extrakorporale Blutbehandlungsvorrichtung zu schaffen, bei der eine unkontrollierte Strömung von Flüssigkeiten im Flüssigkeitssystem vermieden wird.

Die Lösung dieser Aufgaben erfolgt erfindungsgemäß mit den Merkmalen des Patentanspruchs 1. Die abhängigen Ansprüche beziehen sich auf vorteilhafte Ausführungsformen der Erfindung.
Das Flüssigkeitssystem der extrakorporalen Blutbehandlungsvorrichtung, in dem die nicht-okkludierende Pumpe zum Fördern von Flüssigkeit vorgesehen ist, kann der extrakorporale Blutkreislauf oder das Dialysierflüssigkeitssystem der Blutbehandlungsvorrichtung sein. Im extrakorporalen Blutkreislauf soll ein Rückfluss des Bluts des Patienten beim Anhalten oder der Reduzierung der Drehzahl der nicht-okkludierenden Blutpumpe über die Blutzuführleitung vermieden werden. Im Dialysierflüssigkeitssystem kann die Vermeidung einer unkontrollierten Rückwärtsströmung von Dialysierflüssigkeit beim Abschalten oder Reduzieren der Drehzahl der Dialysierflüssigkeitspumpe, insbesondere bei der Verwendung von Flusssensoren zur Bilanzierung von Vorteil sein.

Die erfindungsgemäße Vorrichtung zeichnet sich dadurch aus, dass vor der Reduzierung der Drehzahl oder dem Abschalten der Pumpe die Flüssigkeitsströmung in die Zuführleitung und aus der Abführleitung zunächst unterbrochen wird und nach Ablauf eines vorgegebenen Zeitintervalls nach der Unterbrechung der Flüssigkeitsströmung die Drehzahl der Pumpe reduziert oder die Pumpe abgeschaltet wird. Das zeitverzögerte Abschalten der Pumpe verhindert auch bei fehlender Okklusion der Pumpe ein Rückstrom von Flüssigkeit, insbesondere Blut in die arterielle Blutleitung. Durch das zeitverzögerte Abschalten oder Reduzieren der Drehzahl der Pumpe wird allerdings ein Überdruck im Flüssigkeitssystem erzeugt. Daher sieht die erfindungsgemäße Blutbehandlungsvorrichtung Gegenmaßnahmen vor, um eine unkontrollierte Flüssigkeitsströmung beim Öffnen der Absperrorgane aufgrund des Überdrucks zu verhindern.

Die erfindungsgemäße Blutbehandlungsvorrichtung verfügt über ein erstes Absperrorgan zum Absperren der Zuführleitung und ein zweites Absperrorgan zum Absperren der Abführleitung sowie eine Steuereinheit zum Ansteuern des ersten und zweiten Absperrorgans sowie der nicht-okkludierenden Pumpe. Die Steuereinheit ist derart konfiguriert, dass vor der Reduzierung der Drehzahl oder dem Abschalten der Pumpe das erste und zweite Absperrorgan geschlossen werden und nach Ablauf eines vorgegebenen Zeitintervalls nach dem Schließen des ersten und zweiten Absperrorgans die Drehzahl der Pumpe reduziert oder die Pumpe abgeschaltet wird.

Das vorgegebene Zeitintervall, nach dessen Ablauf die Drehzahl der Pumpe reduziert oder die Pumpe abgeschaltet wird, kann ein fest vorgegebenes Zeitintervall sein, das beispielsweise in einem Speicher der Steuereinheit gespeichert ist. Beispielsweise kann die Pumpe mit einer Zeitverzögerung von 0,5 bis 2 Sekunden, vorzugsweise 0,5 bis 1,5 Sekunden, nach dem Schließen der beiden Absperrorgane zeitverzögert gestoppt werden.

Eine bevorzugte Ausführungsform der Erfindung sieht aber nicht das Reduzieren der Drehzahl oder das Anhalten der Pumpe nach einem fest vorgegebenen Zeitintervall vor, sondern die Drehzahl der Pumpe wird zu einem Zeitpunkt reduziert oder die Pumpe zu einem Zeitpunkt abgeschaltet, zu dem der Durchfluss von Flüssigkeit in der Leitung kleiner als ein vorgegebener Grenzwert ist. Der Grenzwert kann ein Wert größer oder gleich Null sein. Vorzugsweise ist der Grenzwert geringfügig größer oder gleich Null, so dass die Pumpe dann abgeschaltet wird, wenn der Durchfluss in der Leitung nahe dem Nullpunkt ist oder Null ist. In der Praxis wird ein Abschalten der Blutpumpe ausreichend sein, wenn sich der Durchfluss dem Nullpunkt nähert.

Wenn die Pumpe in der Zuführleitung angeordnet ist, wird der Durchfluss in der Zuführleitung gemessen. Hierzu verfügt die erfindungsgemäße Vorrichtung über eine Einrichtung zum Messen des Durchflusses in der Zuführleitung. Vor dem Öffnen des ersten und zweiten Absperrorgans ist für eine kontrollierte Flüssigkeitsströmung der Abbau des Überdrucks erforderlich, der sich im Flüssigkeitssystem aufgebaut hat. Die erfindungsgemäße Vorrichtung sieht hierfür unterschiedliche Gegenmaßnahmen vor.

Bei einer bevorzugten Ausführungsform verfügt die Blutbehandlungsvorrichtung über eine Einrichtung zum Abbau des Überdrucks im Flüssigkeitssystem, wobei die Steuereinheit derart konfiguriert ist, dass nach der Reduzierung der Drehzahl oder dem Abschalten der Pumpe die Einrichtung zum Abbau des Überdrucks vor dem Öffnen des ersten und zweiten Absperrorgans betätigt wird, so dass der Überdruck im Flüssigkeitssystem abgebaut wird. Nach dem Abbau des Überdrucks können die Absperrorgane gleichzeitig oder nacheinander geöffnet werden, ohne dass die Gefahr einer unkontrollierten Flüssigkeitsströmung besteht.

Eine bevorzugte Ausführungsform der Einrichtung sieht einen Abbau des Überdrucks durch ein Entlüftungsventil vor, das von der Steuereinheit betätigt wird. Das Entlüftungsventil ist vorzugsweise an einem Luftabscheider, insbesondere einer Tropfkammer, vorgesehen, die im Allgemeinen in der Blutabführleitung des extrakorporalen Blutkreislaufs angeordnet ist.

Bei einer alternativen Ausführungsform erfolgt der Druckabbau über die semipermeable Membran des Dialysators. Da die bekannten Blutbehandlungsvorrichtungen im Allgemeinen über eine Ultrafiltrationseinrichtung verfügen, kann der Druckabbau vorteilhafterweise dadurch erfolgen, dass die Steuereinheit die Ultrafiltrationseinrichtung derart ansteuert, dass dem Flüssigkeitssystem Flüssigkeit (Ultrafiltrat) entzogen wird, bevor die Absperrorgane geöffnet werden.

Eine weitere bevorzugte Ausführungsform sieht zur Vermeidung eines unkontrollierten Rückflusses vor, zunächst zu einem ersten Zeitpunkt das zweite Absperrorgan in der Abführleitung und erst dann zu einem nachfolgenden zweiten Zeitpunkt das erste Absperrorgan in der Zuführleitung zu öffnen. Im extrakorporalen Blutkreislauf wird durch das Öffnen des venösen Absperrorgans vor dem arteriellen Absperrorgan ein Rückfluss des Bluts in die arterielle Blutleitung sicher vermieden.

Eine unkontrollierte Flüssigkeitsströmung wird bei einer weiteren bevorzugten Ausführungsform dadurch vermieden, dass im Flüssigkeitssystem vor dem Öffnen der Absperrorgane ein negativer Druck aufgebaut wird. Hierzu wird die nicht-okkludierende Pumpe vor dem Öffnen der Absperrorgane eingeschaltet. Die Pumpe wird vorzugsweise mit einer vorgegebenen Drehzahl betrieben, so dass sich der vorgegebene Unterdruck aufbaut. Die Überwachung des Unterdrucks kann mit den bekannten Drucksensoren erfolgen, die ohnehin in dem Flüssigkeitssystem der bekannten Blutbehandlungsvorrichtungen vorhanden sind.

Die einzelnen Verfahrensschritte zum Abbau des Überdrucks im Flüssigkeitssystem können vor der Aufhebung der Unterbrechung der Flüssigkeitsströmung einzeln oder auch in beliebiger Kombination durchgeführt werden.

Im Folgenden werden verschiedene Ausführungsbeispiele der Erfindung unter Bezugnahme auf die Zeichnungen näher erläutert.

Es zeigen:
Fig. 1 die wesentlichen Komponenten einer extrakorporalen Blutbehandlungsvorrichtung in vereinfachter schematischer Darstellung,
Fig. 2 eine alternative Ausführungsform der Blutbehandlungsvorrichtung in vereinfachter schematischer Darstellung,
Fig. 3 die Drücke und der Durchfluss im extrakorporalen Blutkreislauf bei gleichzeitigem Abschalten der nicht-okkludierenden Pumpe und Schließen der Absperrorgane und
Fig. 4 die Drücke und der Durchfluss im extrakorporalen Blutkreislauf beim Abschalten der nicht-okkludierenden Pumpe und Schließen der Absperrorgane.

Die Blutbehandlungsvorrichtung, insbesondere Hämo(dia)filtrationsvorrichtung weist als Blutbehandlungseinheit einen Dialysator 1 auf, der durch eine semipermeable Membran 2 in eine von Blut durchflossene Blutkammer 3 und eine von Dialysierflüssigkeit durchflossene Dialysierflüssigkeitskammer 4 getrennt ist. Zu dem Einlass der Blutkammer 3 führt eine arterielle Blutzuführleitung 5, in die eine Blutpumpe 6 geschaltet ist, während von dem Auslass der Blutkammer 3 eine venöse Blutabführleitung 7 abgeht. Die Blutabführleitung 7 weist einen ersten Abschnitt 7A, der zu dem Einlass 8A eines Luftabscheiders 8, insbesondere eine Tropfkammer, führt, und einen zweiten Abschnitt 7B auf, der von dem Auslass 8B des Luftabscheiders 8 abgeht. Zum Anschluss an den Patienten befinden sich an der Blutzuführ- und -abführleitung 5, 7 eine nicht dargestellte arterielle bzw. venöse Kanüle.

In die Blutzuführleitung 5 ist stromauf der Blutpumpe 6 ein erstes Absperrorgan 9 geschaltet, und in die Blutabführleitung 7 ist stromab des Luftabscheiders 8 ein zweites Absperrorgan 10 geschaltet. Das erste und zweite Absperrorgan 9, 10 können elektromagnetisch oder pneumatisch betätigbare Schlauchklemmen sein.

Die Blutzuführ- und -abführleitung 5, 7 bilden zusammen mit der Blutkammer 3 den extrakorporalen Blutkreislauf I der Blutbehandlungsvorrichtung. Nachfolgend wird das Dialysierflüssigkeitssystem II der Blutbehandlungsvorrichtung beschrieben.

Das Dialysierflüssigkeitssystem II weist eine Dialysierflüssigkeitszuführleitung 11, die von einer Dialysierflüssigkeitsquelle 12 zu der Dialysierflüssigkeitskammer 4 des Dialysators 1 führt, und eine Dialysierflüssigkeitsabführleitung 13 auf, die von der Dialysierflüssigkeitskammer 4 des Dialysators 1 abgeht und zu einem Auslass14 führt.

In die Dialysierflüssigkeitszuführleitung 11 ist eine Kammer 15A einer Bilanziereinrichtung 15 geschaltet, während in die Dialysierflüssigkeitsabführleitung 13 eine andere Kammer 15B der Bilanziereinrichtung 15 zum Bilanzieren von frischer gegen gebrauchte Dialysierflüssigkeit geschaltet ist. In dem Leitungsabschnitt 11A der Dialysierflüssigkeitsabführleitung 13 befindet sich stromauf der Bilanziereinrichtung 15 eine Dialysierflüssigkeitspumpe 16.

Zum Entziehen von Flüssigkeit (Ultrafiltrat) aus dem Blut über die semipermeable Membran 2 des Dialysators 1 weist die Blutbehandlungsvorrichtung eine Ultrafiltrationseinrichtung 17 auf. Die Ultrafiltrationseinrichtung 17 weist eine von der Dialysierflüssigkeitsabführleitung 13 stromauf der Bilanziereinrichtung 15 abzweigende und direkt zu dem Auslass 14 führende Flüssigkeitsleitung 18 auf, in der sich eine Ultrafiltratpumpe 19 befindet.

Darüber hinaus weist die Blutbehandlungsvorrichtung eine zentrale Steuereinheit 20 auf. Die Blutpumpe 6 Dialysierflüssigkeitspumpe 16 und die Ultrafiltratpumpe 19 sowie das erste und zweite Absperrorgan 9, 10 sind über Steuerleitungen 6', 16', 19', 9', 10' mit einer zentralen Steuereinheit 20 verbunden, so dass die Steuereinheit 20 die Pumpen mit einer vorgegebenen Drehzahl betreiben und das erste und zweite Absperrorgan 9, 10 öffnen oder schließen kann.

Des Weiteren verfügt die Blutbehandlungsvorrichtung zum Messen der Drücke im extrakorporalen Blutkreislauf I über eine Druckmesseinrichtung 21, die einen ersten Sensor 21A zum Messen des arteriellen Drucks pₐᵣₜ in der Blutzuführleitung 5 stromauf der Blutpumpe 6, einen zweiten Sensor 21B zum Messen des Drucks pₚᵣₑ in der Blutzuführleitung 5 stromab der Blutpumpe 6 und stromauf des Dialysators 1 und einen dritten Sensor 21C zum Messen des venösen Drucks pᵥₑₙ in der Blutabführleitung 7 stromab des Luftabscheiders 8 aufweist, die über Signalleitungen 21A', 21B', 21' mit der Druckmesseinrichtung 21 verbunden sind. Die Druckmesseinrichtung 21 sendet die Drucksignale über eine Datenleitung 21' an die zentrale Steuereinheit 20.

Zum Messen des Durchflusses Q_{b} an Blut verfügt die Blutbehandlungsvorrichtung über eine Durchflussmesseinrichtung 23, die einen Sensor 23A in der Blutzuführleitung 5 stromauf der Blutpumpe 6 aufweist, der über eine Signalleitung 23A' mit der Durchflussmesseinrichtung verbunden ist. Die Durchflussmesseinrichtung 23 ist über eine Signalleitung 23' mit der zentralen Steuereinheit 20 verbunden.

Die Blutbehandlungsvorrichtung kann noch über weitere Komponenten verfügen, die aber der besseren Übersichtlichkeit halber nicht dargestellt sind. Für die Erfindung ist es aber nicht erforderlich, dass die Blutbehandlungsvorrichtung sämtliche der oben beschriebenen Sensoren aufweist.

Die zentrale Steuereinheit 20 ist derart konfiguriert, dass die einzelnen Komponenten der Blutbehandlungsvorrichtung so angesteuert werden, dass die für die Erfindung erforderlichen Schritte durchgeführt werden. Die Steuereinheit 20 kann hierfür über eine Datenverarbeitungseinheit (Mikroprozessor) verfügen, auf dem ein Datenverarbeitungsprogramm (Software) läuft.

Bei der Blutpumpe 6 handelt es sich um eine nicht-okkludierende Blutpumpe, die nicht über einen Verschlusskörper zum Verschließen der Schlauchleitung verfügt. Die nicht-okkludierende Pumpe kann beispielsweise eine Zentrifugal- oder Impellerpumpe sein.

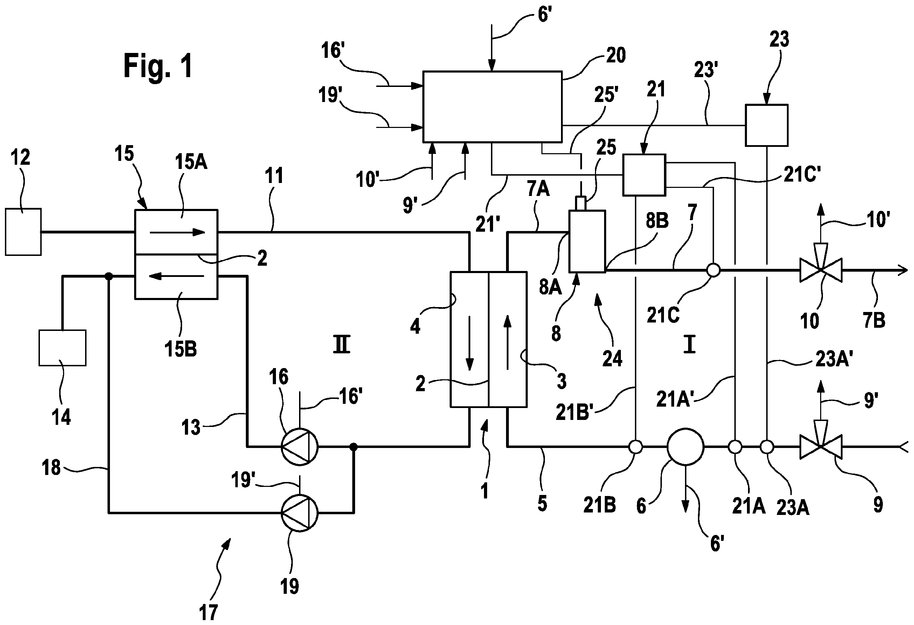

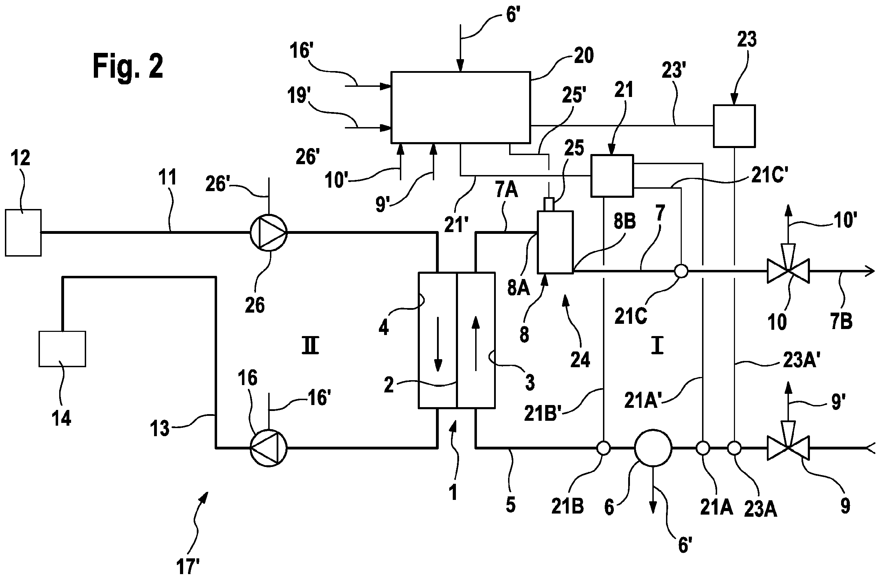

Fig. 2 zeigt eine alternative Ausführungsform der Blutbehandlungsvorrichtung, die sich von dem Ausführungsbeispiel von Fig. 1 nur dadurch unterscheidet, dass die Bilanzierung von frischer gegen verbrauchte Dialysierflüssigkeit durch Einstellung der Förderraten der in der Dialysierflüssigkeitsabführleitung 13 angeordneten Dialysierflüssigkeitspumpe 16 und einer in der Dialysierflüssigkeitszuführleitung 11 angeordneten Dialysierflüssigkeitspumpe 26 erfolgt, die über eine Steuerleitung 26' mit der zentralen Steuereinheit 20 verbunden ist. Durch Einstellung unterschiedlicher Förderraten beider Pumpen 16, 26 kann dem Dialysierflüssigkeitssystem eine vorgegebene Menge an die Dialysierflüssigkeit entzogen werden (Ultrafiltration). Bei der alternativen Ausführungsform ist eine separate Ultrafiltrationspumpe nicht vorgesehen. Es sind auch keine Bilanzkammern vorgesehen. Die Ultrafiltrationseinrichtung 17' der alternativen Ausführungsform umfasst die in der Dialysierflüssigkeitsabführleitung 13 angeordnete Dialysierflüssigkeitspumpe 16 und die in der Dialysierflüssigkeitszuführleitung 11 angeordnete Dialysierflüssigkeitspumpe 26. Die einander entsprechenden Teile sind in den Figuren 1 und 2 mit den gleichen Bezugszeichen versehen.

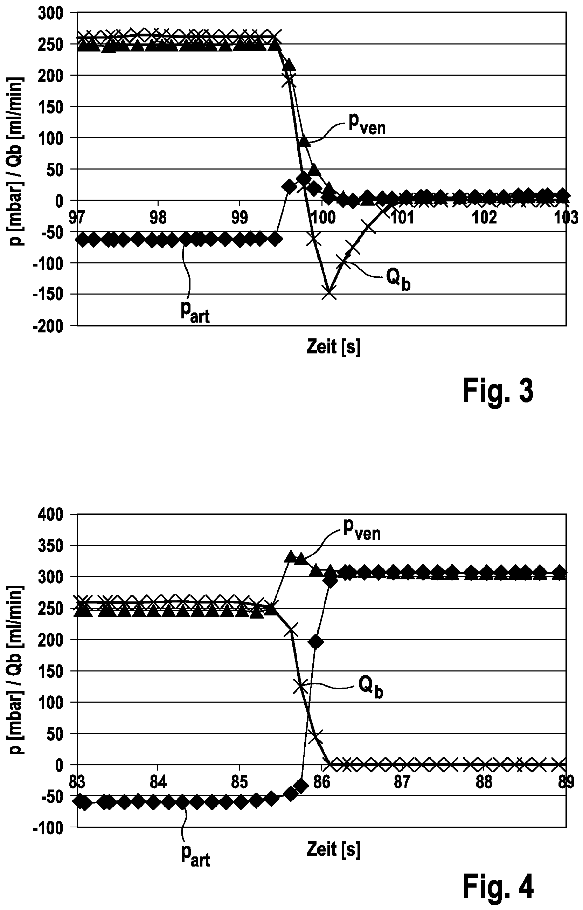

Die Steuerung der Blutbehandlungsvorrichtung sieht einen Steuermodus zum Abschalten der Blutpumpe 6 oder zur Reduzierung deren Drehzahl vor. In Versuchen hat sich gezeigt, dass beim gleichzeitigen Schließen des ersten und zweiten Absperrorgans 9, 10 und Anhalten der Blutpumpe 6 Blut durch die arterielle Blutzuführleitung 5 zurück zum Patienten strömt, obwohl das arterielle Absperrorgan 9 beim Abschalten der Blutpumpe 6 geschlossen wird. Fig. 3 zeigt die Druckverhältnisse im extrakorporalen Blutkreislauf, wenn gleichzeitig das erste und zweite Absperrorgan 9, 10 geschlossen und die Blutpumpe 6 gestoppt werden. In Fig. 3 ist der mit dem ersten Sensor 21A stromauf der Blutpumpe 6 gemessene arterielle Druck mit pₐᵣₜ, der mit dem zweiten Sensor 21B gemessene Druck mit pₚᵣₑ, und der mit dem dritten Sensor 21C gemessene venöse Druck mit pᵥₑₙ bezeichnet. Der mit dem Sensor 23A stromauf der Blutpumpe 6 in der Blutzuführleitung 5 gemessene Blutfluss ist in Fig. 3 mit Q_{b} bezeichnet.

Es hat sich in den Versuchen gezeigt, dass das erste und zweite Absperrorgan 9, 10 eine gewisse Zeit brauchen, um die Schlauchleitungen abzuklemmen, weshalb der Druck in den Leitungen nicht sprunghaft abnimmt. Während die Schlauchklemmen schließen, kommt es zu einem Rückfluss von Blut in der Blutzuführleitung 5 in Richtung des Patienten. Die Rückflussrate betrug bei der Messung bis zu ca. 150 ml/min, was etwa die Hälfte der Flussrate in die andere Richtung zum Dialysator 1 entspricht.

Nachfolgend wird im Einzelnen beschrieben, wie die zentrale Steuereinheit 20 der erfindungsgemäßen Blutbehandlungsvorrichtung in dem Steuermodus zum Anhalten oder Reduzieren der Drehzahl der Blutpumpe 6 die einzelnen Komponenten der Blutbehandlungsvorrichtung zur Verhinderung eines unkontrollierten Rückflusses zum Patienten ansteuert.

Zunächst schließt die Steuereinheit 20 gleichzeitig das erste und zweite Absperrorgan 9, 10 zu einem Zeitpunkt t₁. Erst nach einem vorgegebenen Zeitintervall Δt zu einem Zeitpunkt t₂ hält die Steuereinheit 20 die Blutpumpe 6 an oder reduziert deren Drehzahl. Das zeitverzögerte Anhalten der Blutpumpe 6 oder Reduzieren der Drehzahl hat zur Folge, dass sich im extrakorporalen Blutkreislauf I ein Überdruck aufbaut, so dass ein Rückfluss zum Patienten nicht stattfinden kann. Bei dem vorliegenden Ausführungsbeispiel ist das Zeitintervall Δt ein fest vorgegebenes Zeitintervall. Die Zeitverzögerung zum Stoppen der Pumpe 6 kann beispielsweise ein in einem Speicher der Steuereinheit abgelegter Wert sein.

Fig. 4 zeigt die Druck- und Flussverhältnisse beim Stoppen der Blutpumpe 6 und Schließen der Absperrorgane 9, 10. Es zeigt sich der Aufbau des Überdrucks im extrakorporalen Blutkreislauf I. Der arterielle Blutfluss Q_{b} fällt von ca. 250 ml/min auf Null ab, während der Druck pₐᵣₜ, pₚᵣₑ, pᵥₑₙ im Blutkreislauf I auf ca. 300 mbar ansteigt. Es zeigt sich ein Abfallen des Blutflusses auf Null, ohne dass ein Rückfluss stattfindet. Bei dem in Fig. 4 gezeigten Ausführungsbeispiel wurde die Blutpumpe 6 ca. 1,2 Sekunden nach dem Schließen der Absperrorgane 9, 10 gestoppt.

Eine Ausführungsform der Erfindung sieht ein Stoppen der Blutpumpe 6 nicht nach einem fest vorgegebenen Zeitintervall Δt vor, sondern zu einem Zeitpunkt t₃, zu dem sich der Blutfluss Q_{b} dem Nullpunkt nähert oder Null wird. Bei dieser Ausführungsform empfängt die Steuereinheit 20 das von der Durchflussmesseinrichtung 23 gemessene Messsignal, wobei die Steuereinheit den gemessenen Blutfluss Q_{b} mit einem vorgegebenen Grenzwert vergleicht, der um einen bestimmten, vorzugsweise geringfügigen Wert größer als Null oder gleich Null ist. Wenn der Durchfluss Q_{b} zu dem Zeitpunkt t₃ gleich dem Grenzwert ist, hält die Steuereinheit 20 die Blutpumpe 6 an oder reduziert deren Drehzahl.

Wenn ein unkontrollierter Blutfluss auch beim Wiedereinschalten der Blutpumpe 6 oder der Erhöhung der Drehzahl verhindert werden soll, ist ein Abbau des Überdrucks im Blutkreislauf I erforderlich. Zum Abbau des Überdrucks ist eine Einrichtung 24 vorgesehen, die verschiedene Komponenten umfassen kann, die in einer Blutbehandlungsvorrichtung ohnehin bereits vorhanden sind.

Bei einer Ausführungsform setzt die Steuereinheit 20 zum Abbau des Überdrucks vor dem Öffnen des ersten und zweiten Absperrorgans 9, 10 die Ultrafiltrationspumpe 19 der Ultrafiltrationseinrichtung 17 für ein vorgegebenes Zeitintervall in Betrieb, so dass sich der Überdruck über die semipermeable Membran 2 des Dialysators 1 abbaut (Fig. 1). Eine andere Ausführungsform sieht zum Abbau des Überdrucks vor dem Öffnen der Absperrorgane 9, 10 ein an dem Luftabscheider 8 vorgesehenes Entlüftungsventil 25 vor, das über eine Steuerleitung 25' mit der Steuereinheit 20 verbunden ist. Die Steuereinheit 20 öffnet zunächst das Entlüftungsventil 25 für ein vorgegebenes Zeitintervall Δt und öffnet dann erst das erste und zweite Absperrorgan 9, 10.

Bei der alternativen Ausführungsform von Fig. 2 stellt die Steuereinheit 20 zum Abbau des Überdrucks für die in der Dialysierflüssigkeitsabführleitung 13 angeordnete Dialysierflüssigkeitspumpe 16 und die in der Dialysierflüssigkeitszuführleitung 11 angeordnete Dialysierflüssigkeitspumpe 26 unterschiedliche Förderraten ein, wobei die in der Dialysierflüssigkeitsabführleitung angeordnete Dialysierflüssigkeitspumpe 16 mit einer größeren Förderrate (Drehzahl) betrieben wird, als die in der Dialysierflüssigkeitszuführleitung 11 angeordnete Dialysierflüssigkeitspumpe 26, so dass dem Flüssigkeitssystem Flüssigkeit (Ultrafiltrat) entzogen wird.

Bei einer weiteren Ausführungsform setzt die Steuereinheit 20 zum Abbau des Überdrucks vor dem Öffnen des ersten und zweiten Absperrorgans 9, 10 die Blutpumpe 6 für ein vorgegebenes Zeitintervall Δt in Betrieb. Vorzugsweise wird die Blutpumpe 6 mit einer vorgegebenen Drehzahl n solange betrieben, bis sich ein vorgegebener Unterdruck pₐᵣₜ in der arteriellen Blutzuführleitung 5 stromauf der Pumpe einstellt. Der Unterdruck wird mit dem Sensor 21B der Druckmesseinrichtung 21 überwacht, wobei die Steuereinheit 20 die von der Druckmesseinrichtung gemessenen Drucksignale empfängt und das erste und zweite Absperrorgan nach Erreichen des erforderlichen Unterdrucks öffnet. Der Aufbau des Unterdrucks im Blutkreislauf I kann als zusätzliche Maßnahme auch mit den anderen Verfahren vorteilhaft kombiniert werden.

Eine weitere Ausführungsform sieht vor, dass die Steuereinheit 20 zu einem ersten Zeitpunkt t₁ zunächst das zweite Absperrorgan 10 in der venösen Blutabführleitung 7 öffnet und erst zu einem in einem vorgegebenen Zeitintervall Δt nachfolgenden Zeitpunkt t₂ das erste Absperrorgan in der arteriellen Blutzuführleitung 5 öffnet. Dadurch kann Blut nur über die venöse Leitung 5 in den Patienten, nicht aber über die arterielle Leitung 7 zurück zum Patienten strömen.

## Patentansprüche

1. Extrakorporale Blutbehandlungsvorrichtung mit
einem Flüssigkeitssystem (I), das eine zu einer Blutbehandlungseinheit (1) führende Zuführleitung (5) und eine von der Blutbehandlungseinheit abgehende Abführleitung (7) aufweist, wobei die Blutbehandlungseinheit (1) durch eine semipermeable Membran (2) in eine Blutkammer (3) und eine Dialysierflüssigkeitskammer (4) unterteilt ist,
einer Pumpe (6) zum Fördern von Flüssigkeit in der Zuführ- oder Abführleitung (5, 7) des Flüssigkeitssystems (I), wobei die Pumpe eine nicht-okkludierende Pumpe ist,
einem ersten Absperrorgan (9) zum Absperren der Zuführleitung (5) und einem zweiten Absperrorgan (10) zum Absperren der Abführleitung (7) und
einer Steuereinheit (20) zum Ansteuern des ersten und zweiten Absperrorgans (9, 10) und der nicht-okkludierenden Pumpe (6),
**dadurch gekennzeichnet, dass**
die Steuereinheit (20) derart konfiguriert ist, dass vor der Reduzierung der Drehzahl oder dem Abschalten der Pumpe das erste und zweite Absperrorgan (9, 10) geschlossen werden und nach Ablauf eines vorgegebenen Zeitintervalls nach dem Schließen des ersten und zweiten Absperrorgans die Drehzahl der Pumpe (6) reduziert oder die Pumpe abgeschaltet wird.

2. Blutbehandlungsvorrichtung nach Anspruch 1, **dadurch gekennzeichnet, dass** das Flüssigkeitssystem der extrakorporale Blutkreislauf (I) der Blutbehandlungsvorrichtung ist, wobei die Zuführleitung (5) an einen Einlass der Blutkammer (3) und die Abführleitung (7) an einen Auslass der Blutkammer (3) angeschlossen ist und die Pumpe (6) in der Zuführleitung (5) angeordnet ist.

3. Blutbehandlungsvorrichtung nach Anspruch 1 oder 2, **dadurch gekennzeichnet, dass** eine Einrichtung (23) zum Messen des Durchflusses in der Zuführleitung (5) vorgesehen ist, und die Steuereinheit (20) derart konfiguriert ist, dass die Drehzahl der Pumpe (6) zu einem Zeitpunkt reduziert oder die Pumpe zu einem Zeitpunkt abgeschaltet wird, zu dem der in der Zuführleitung (5) gemessene Durchfluss kleiner oder gleich als ein vorgegebener Grenzwert ist, der vorzugsweise Null ist.

4. Blutbehandlungsvorrichtung nach einem der Ansprüche 1 bis 3, **dadurch gekennzeichnet, dass** eine Einrichtung (24) zum Abbau eines Überdrucks im Flüssigkeitssystem (I) vorgesehen ist, wobei die Steuereinheit (20) derart konfiguriert ist, dass nach der Reduzierung der Drehzahl oder dem Abschalten der Pumpe (8) die Einrichtung (24) zum Abbau eines Überdrucks vor dem Öffnen des ersten und zweiten Absperrorgans (9, 10) betätigt wird, so dass der Überdruck im Flüssigkeitssystem (I) abgebaut wird.

5. Blutbehandlungsvorrichtung nach Anspruch 4, **dadurch gekennzeichnet, dass** die Einrichtung (24) zum Abbau eines Überdrucks ein von der Steuereinheit (20) betätigbares Entlüftungsventil (25) aufweist.

6. Blutbehandlungsvorrichtung nach Anspruch 5, **dadurch gekennzeichnet, dass** in der Abführleitung (7) ein Luftabscheider (8) angeordnet ist, wobei das Entlüftungsventil (25) an dem Luftabscheider vorgesehen ist.

7. Blutbehandlungsvorrichtung nach Anspruch 4, **dadurch gekennzeichnet, dass**
die Blutbehandlungsvorrichtung eine Ultrafiltrationseinrichtung (17) aufweist, die eine von der Steuereinheit (20) betätigbare Ultrafiltrationspumpe (19) aufweist, wobei die Einrichtung (24) zum Abbau eines Überdrucks die Ultrafiltrationspumpe (19) der Ultrafiltrationseinrichtung (17) umfasst, oder
die Blutbehandlungsvorrichtung eine Ultrafiltrationseinrichtung (17') aufweist, die eine in der Dialysierflüssigkeitsabführleitung (13) angeordnete Dialysierflüssigkeitspumpe (16) und eine in der Dialysierflüssigkeitszuführleitung (11) angeordnete Dialysierflüssigkeitspumpe (26) aufweist, die von der Steuereinheit (20) betätigbar sind, wobei die Einrichtung (24) zum Abbau eines Überdrucks die in der Dialysierflüssigkeitsabführleitung (13) angeordnete Dialysierflüssigkeitspumpe (16) und die in der Dialysierflüssigkeitszuführleitung (11) angeordnete Dialysierflüssigkeitspumpe (26) umfasst.

8. Blutbehandlungsvorrichtung nach Anspruch 1 oder 2, **dadurch gekennzeichnet, dass** die Steuereinheit (20) derart konfiguriert ist, dass nach der Reduzierung der Drehzahl oder dem Abschalten der Pumpe (6) und vor dem Einschalten der Pumpe oder der Erhöhung der Drehzahl der Pumpe zu einem ersten Zeitpunkt das zweite Absperrorgan (10) in der Abführleitung (7) und zu einem nachfolgenden zweiten Zeitpunkt das erste Absperrorgan (9) in der Zuführleitung (7) geöffnet werden.

9. Blutbehandlungsvorrichtung nach einem der Ansprüche 1 bis 8, **dadurch gekennzeichnet, dass** die Steuereinheit (20) derart konfiguriert ist, dass nach der Reduzierung der Drehzahl oder dem Abschalten der Pumpe (6) die Pumpe vor dem Öffnen des ersten und zweiten Absperrorgans (9, 10) eingeschaltet oder die Drehzahl der Pumpe erhöht wird.

10. Blutbehandlungsvorrichtung nach Anspruch 9, **dadurch gekennzeichnet, dass** die Steuereinheit (20) derart konfiguriert ist, dass die Pumpe mit einer Drehzahl betrieben wird, so dass sich in der Zuführleitung (5) ein vorgegebener Unterdruck aufbaut.

## Claims

1. Extracorporeal blood treatment apparatus having
a fluid system (I) that has a feed line (5) that leads to a blood treatment unit (1) and a discharge line (7) that leads away from the blood treatment unit, wherein the blood treatment unit (1) is divided into a blood chamber (3) and a dialysis fluid chamber (4) by means of a semi-permeable membrane (2),
a pump (6) for conveying fluid in the feed line or the discharge line (5, 7) of the fluid system (I), wherein the pump is a non-occluding pump,
a first shut-off element (9) for shutting off the feed line (5) and a second shut-off element (10) for shutting off the discharge line (7), and
a control unit (20) for controlling the first and the second shut-off element (9, 10) and the non-occluding pump (6),
**characterized in that**
the control unit (20) is configured in such a manner that before the speed of rotation of the pump is reduced or the pump is shut off, the first and the second shut-off element (9, 10) are closed, and the speed of rotation of the pump (6) is reduced or the pump is shut off after a predetermined time interval after the first and the second shut-off element were closed has elapsed.

2. Blood treatment apparatus according to claim 1, **characterized in that** the fluid system is the extra corporeal blood circuit (I) of the blood treatment apparatus, wherein the feed line (5) is connected with an inlet of the blood chamber (3) and the discharge line (7) is connected with an outlet of the blood chamber (3), and the pump (6) is disposed in the feed line (5).

3. Blood treatment apparatus according to claim 1 or 2, **characterized in that** a device (23) for measuring the flow through the feed line (5) is provided, and the control unit (20) is configured in such a manner that the speed of rotation of the pump (6) is reduced at a point in time or the pump is shut off at a point in time when the through-flow measured in the feed line (5) is less than or equal to a predetermined limit value, which is preferably zero.

4. Blood treatment apparatus according to one of claims 1 to 3, **characterized in that** a device (24) for reducing an excess pressure in the fluid system (I) is provided, wherein the control unit (20) is configured in such a manner that after the speed of rotation is reduced or the pump (8) is shut off, the device (24) for reducing an excess pressure is activated before the first and the second shut-off element (9, 10) are opened, so that the excess pressure in the fluid system (I) is reduced.

5. Blood treatment apparatus according to claim 4, **characterized in that** the device (24) for reducing an excess pressure has a ventilation valve (25) that can be activated by the control unit (20).

6. Blood treatment apparatus according to claim 5, **characterized in that** an air separator (8) is disposed in the discharge line (7), wherein the ventilation valve (25) is provided on the air separator.

7. Blood treatment apparatus according to claim 4, **characterized in that**
the blood treatment apparatus has an ultrafiltration device (17) that has an ultrafiltration pump (19) that can be activated by the control unit (20), wherein the device (24) for reducing an excess pressure comprises the ultrafiltration pump (19) of the ultrafiltration device (17), or
the blood treatment apparatus has an ultrafiltration device (17') that has a dialysis fluid pump (16) disposed in the dialysis fluid discharge line (13) and a dialysis fluid pump (26) disposed in the dialysis fluid feed line (11), which can be activated by the control unit (20), wherein the device (24) for reducing an excess pressure comprises the dialysis fluid pump (16) disposed in the dialysis fluid discharge line (13) and the dialysis fluid pump (26) disposed in the dialysis fluid feed line (11).

8. Blood treatment apparatus according to claim 1 or 2, **characterized in that** the control unit (20) is configured in such a manner that after the speed of rotation is reduced or the pump (6) is shut off, and before the pump is turned on or the speed of rotation of the pump is increased, the second shut-off element (10) in the discharge line (7) is opened at a first point in time, and the first shut-off element (9) in the feed line (7) is opened at a subsequent second point in time.

9. Blood treatment apparatus according to one of claims 1 to 8, **characterized in that** the control unit (20) is configured in such a manner that after the speed of rotation is reduced or the pump (6) is shut off, the pump is turned on before the first and the second shut-off element (9, 10) are opened or the speed of rotation of the pump is increased.

10. Blood treatment apparatus according to claim 9, **characterized in that** the control unit (20) is configured in such a manner that the pump is operated at a speed of rotation so that a predetermined partial vacuum builds up in the feed line (5).

## Revendications

1. Dispositif de traitement du sang extracorporel avec
un système à liquide (I), qui présente une conduite d'amenée (5) menant à une unité de traitement du sang (1) et une conduite d'évacuation (7) partant de l'unité de traitement du sang, où l'unité de traitement du sang (1) est subdivisée par une membrane semi-perméable (2) en une chambre à sang (3) et une chambre à liquide de dialyse (4),
une pompe (6) pour le transport de liquide dans la conduite d'amenée ou d'évacuation (5, 7) du système à liquide (I), où la pompe est une pompe non occlusive,
un premier organe d'obturation (9) pour l'obturation de la conduite d'amenée (5) et un second organe d'obturation (10) pour l'obturation de la conduite d'évacuation (7) et
une unité de commande (20) pour la commande du premier et du second organe d'obturation (9, 10) et de la pompe non occlusive (6),
**caractérisé en ce que**
l'unité de commande (20) est configurée de telle façon qu'avant la réduction de la vitesse de rotation ou la mise hors service de la pompe, le premier et le second organe d'obturation (9, 10) sont fermés et après l'écoulement d'un intervalle de temps prédéterminé après la fermeture du premier et du second organe d'obturation, la vitesse de rotation de la pompe (6) est réduite ou la pompe est mise hors service.

2. Dispositif de traitement du sang selon la revendication 1, **caractérisé en ce que** le système à liquide est le circuit de sang extracorporel (I) du dispositif de traitement du sang, où la conduite d'amenée (5) est raccordée à une entrée de la chambre à sang (3) et la conduite d'évacuation (7) est raccordée à une sortie de la chambre à sang (3) et la pompe (6) est disposée dans la conduite d'amenée (5).

3. Dispositif de traitement du sang selon la revendication 1 ou 2, **caractérisé en ce qu'**une installation (23) pour la mesure de l'écoulement dans la conduite d'amenée (5) est prévue, et l'unité de commande (20) est configurée de telle façon que la vitesse de rotation de la pompe (6) est réduite à un moment, ou la pompe est mise hors service à un moment, auquel l'écoulement mesuré dans la conduite d'amenée (5) est inférieur ou égal à une valeur limite prédéterminée, qui est de préférence égale à zéro,

4. Dispositif de traitement du sang selon l'une des revendications 1 à 3,
**caractérisé en ce qu'**une installation (24) pour le relâchement d'une surpression dans le système à liquide (I) est prévue, où l'unité de commande (20) est configurée de telle façon qu'après la réduction de la vitesse de rotation ou la mise hors service de la pompe (8), l'installation (24) pour le relâchement d'une surpression est actionnée avant l'ouverture du premier et du second organe d'obturation (9, 10), de sorte que la surpression dans le système à liquide (1) est relâchée.

5. Dispositif de traitement du sang selon la revendication 4, **caractérisé en ce que** l'installation (24) pour le relâchement d'une surpression présente une soupape d'évacuation d'air (25) qui peut être actionnée par l'unité de commande (20).

6. Dispositif de traitement du sang selon la revendication 5, **caractérisé en ce qu'**un séparateur d'air (8) est disposé dans la conduite d'évacuation (7), où la soupape d'évacuation d'air (25) est prévue sur le séparateur d'air.

7. Dispositif de traitement du sang selon la revendication 4, **caractérisé en ce que** le dispositif de traitement du sang présente une installation d'ultrafiltration (17) qui présente une pompe d'ultrafiltration (19) qui peut être actionnée par l'unité de commande (20), où l'installation (24) pour le relâchement d'une surpression comprend la pompe d'ultrafiltration (19) de l'installation d'ultrafiltration (17), ou
le dispositif de traitement du sang présente une installation d'ultrafiltration (17') qui présente une pompe à liquide de dialyse (16) disposée dans la conduite d'évacuation de liquide de dialyse (13) et une pompe à liquide de dialyse (26) disposée dans conduite d'amenée de liquide de dialyse (11), qui peuvent être actionnées par l'unité de commande (20), où l'installation (24) pour le relâchement d'une surpression comprend la pompe à liquide de dialyse (16) disposée dans la conduite d'évacuation de liquide de dialyse (13) et la pompe à liquide de dialyse (26) disposée dans la conduite d'amenée de liquide de dialyse (11).

8. Dispositif de traitement du sang selon la revendication 1 ou 2, **caractérisé en ce que** l'unité de commande (20) est configurée de telle façon qu'après la réduction de la vitesse de rotation ou la mise hors service de la pompe (6) et avant la mise en service de la pompe ou l'augmentation de la vitesse de rotation de la pompe à un premier moment le second organe d'obturation (10) dans la conduite d'évacuation (7) et à un second moment consécutif le premier organe d'obturation (9) dans la conduite d'amenée (7) sont ouverts.

9. Dispositif de traitement du sang selon l'une des revendications 1 à 8,
**caractérisé en ce que** l'unité de commande (20) est configurée de telle façon qu'après la réduction de la vitesse de rotation ou la mise hors service de la pompe (6), la pompe est mise en service avant l'ouverture du premier et du second organe d'obturation (9, 10) ou la vitesse de rotation de la pompe est augmentée.

10. Dispositif de traitement du sang selon la revendication 9, **caractérisé en ce que** l'unité de commande (20) est configurée de telle façon que la pompe est amenée à fonctionner avec une vitesse de rotation de sorte qu'une dépression prédéterminée s'établit dans la conduite d'amenée (5).
